(19) Europäisches Patentamt
European Patent Office
Office européen des brevets

(11) **EP 3 579 193 B1**

(12) **EUROPÄISCHE PATENTSCHRIFT**

(45) Veröffentlichungstag und Bekanntmachung des Hinweises auf die Patenterteilung:
**23.12.2020 Patentblatt 2020/52**

(51) Int Cl.:
**G06T 11/00** *(2006.01)*

(21) Anmeldenummer: **18176803.7**

(22) Anmeldetag: **08.06.2018**

(54) **VERFAHREN ZUR ERMITTLUNG VON BILDWERTEN IN MARKIERTEN PIXELN WENIGSTENS EINES PROJEKTIONSBILDES, RÖNTGENEINRICHTUNG, COMPUTERPROGRAMM UND ELEKTRONISCH LESBARER DATENTRÄGER**

METHOD FOR DETERMINING IMAGE VALUES IN MARKED PIXELS OF AT LEAST ONE PROJECTION IMAGE, X-RAY DEVICE, COMPUTER PROGRAM AND ELECTRONICALLY READABLE DATA CARRIER

PROCÉDÉ DE DÉTERMINATION DES VALEURS D'IMAGE EN PIXELS MARQUÉS D'AU MOINS UNE IMAGE DE PROJECTION, DISPOSITIF À RAYONS X, PROGRAMME INFORMATIQUE ET SUPPORT DE DONNÉES LISIBLE ÉLECTRONIQUEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**11.12.2019 Patentblatt 2019/50**

(73) Patentinhaber: **Siemens Healthcare GmbH**
**91052 Erlangen (DE)**

(72) Erfinder: **Manhart, Michael**
**90765 Fürth (DE)**

(56) Entgegenhaltungen:
**DE-B3-102017 200 282**

- **Tobias Würfl ET AL: "A new calibration-free beam hardening reduction method for industrial CT", 8th Conference on Industrial Computed Tomography, Februar 2018 (2018-02), Seiten 1-7, XP055522972, Gefunden im Internet: URL:https://www.ndt.net/article/ctc2018/pa pers/ICT2018_paper_id153.pdf [gefunden am 2018-11-12]**
- **ANDRE AICHERT ET AL.: "Epipolar Consistency in Transmission Imaging", IEEE TRANSACTIONS ON MEDICAL IMAGING, Bd. 34, 2015, Seiten 2205-2219, XP011588380,**
- **PREUHS ALEXANDER ET AL: "Fast Epipolar Consistency without the Need for Pseudo Matrix Inverses", ARXIV.ORG, CORNELL UNIVERSITY LIBRARY, 201 OLIN LIBRARY CORNELL UNIVERSITY ITHACA, NY 14853, 7. Juni 2018 (2018-06-07), XP080888134,**

EP 3 579 193 B1

**Beschreibung**

Die Erfindung betrifft ein Verfahren zur Ermittlung von Bildwerten in markierten Pixeln wenigstens eines Projektionsbildes, wobei das wenigstens eine Projektionsbild Teil eines in einem Aufnahmevorgang jeweils in einer Projektionsgeometrie aufgenommene Projektionsbilder umfassenden, zur Rekonstruktion eines dreidimensionalen Bilddatensatzes vorgesehenen Projektionsbildersatzes ist. Daneben betrifft die Erfindung eine Röntgeneinrichtung, ein Computerprogramm und einen elektronisch lesbaren Datenträger.

Insbesondere in der medizinischen Röntgenbildgebung ist es inzwischen gängig, dreidimensionale Bilddatensätze eines Untersuchungsbereichs eines Patienten zu erzeugen, indem zunächst unter Nutzung unterschiedlicher Projektionsgeometrien Röntgen-Projektionsbilder, beispielsweise entlang einer Kreistrajektorie, aufgenommen werden. Diese zweidimensionalen Röntgenprojektionsbilder können zur Rekonstruktion eines dreidimensionalen Bilddatensatzes des Untersuchungsbereichs herangezogen werden, beispielsweise unter Verwendung der Methode der gefilterten Rückprojektion und/oder der iterativen (algebraischen) Rekonstruktion. Beispielsweise können dabei 200 bis 500 Projektionsbilder unter Nutzung unterschiedlicher Projektionsgeometrien, insbesondere bei einer Kreisbahn um den Untersuchungsbereich des Patienten Projektionswinkeln, aufgenommen werden, die beispielsweise ein Winkelintervall von 180° plus dem Fächerwinkel abdecken können. Ein entsprechendes Vorgehen wird beispielsweise in der Computertomographie eingesetzt.

Das Vorliegen von Metallen im Untersuchungsbereich kann die Bildqualität des dreidimensionalen Bilddatensatzes deutlich einschränken. Beispielsweise können sogenannte Metallartefakte auftreten. Zur Verbesserung der Bildqualität und zur Beseitigung von Metallartefakten wurde vorgeschlagen, die metallischen Objekte im Untersuchungsbereich aus den Projektionsdaten des Projektionsbildersatzes herauszurechnen, das bedeutet, die Metall anzeigenden Bildwerte durch Bildwerte zu ersetzen, wie sie (näherungsweise) ohne Vorliegen des entsprechenden Metallobjekts im Untersuchungsbereich dort erwartet würden. Ein solches Vorgehen wird auch Metallartefaktreduktion (MAR) genannt.

Zur Metallartefaktreduktion sind im Stand der Technik bereits verschiedene Vorgehensweisen bekannt. So wurde beispielsweise vorgeschlagen, innerhalb der Projektionsbilder eine Interpolation über die Metall zeigenden Bildbereiche vorzunehmen. Allerdings ist eine derartige Interpolation insbesondere bei trunkierten Volumina, beispielsweise bei Aufnahme der Projektionsbilder mit einer Röntgeneinrichtung mit einem C-Bogen, ungenau, was zu einer weiterhin eingeschränkten Bildqualität führt.

In einem Artikel von Esther Meyer et al., "Normalized metal artifact reduction (NMAR) in computed tomography", Medical Physics 37, 5482 (2010), wurde die sogenannte normalisierte Metallartefaktreduktion eingeführt, bei der Metallobjekte im dreidimensionalen Bilddatensatz, beispielsweise durch ein Schwellwertverfahren, segmentiert werden. Durch eine Vorwärtsproduktion können die Metall zeigenden Pixel in den Projektionsbildern identifiziert werden. Vor der Interpolation werden die Projektionsbilder basierend auf einer 3D-Vorwärtsprojektion eines Vorabbildes normalisiert, welches durch eine Mehrgrenzwert-Segmentierung des ursprünglichen Bildes erzeugt werden kann. Die ursprünglichen Rohdaten werden durch die Projektionsdaten des Vorabbildes dividiert und nach der Interpolation wieder denormalisiert. Allerdings benötigt die normalisierte Metallartefaktreduktion eine lateral nicht trunkierte Aufnahme des Objekts für den normalisierten Interpolationsschritt, da dabei die zu interpolierenden Werte aus numerisch projizierten Strahlen durch den Untersuchungsbereich geschätzt werden.

Neben derartigen Interpolationsverfahren wurden auch auf künstlicher Intelligenz basierende Verfahren zur Schätzung fehlender Bildwerte in Betracht gezogen, wie dies beispielsweise zur Schätzung von Maskenbildern in der koronaren DSA-Bildgebung in einem Artikel von Mathias Unberath et al., "Deep Learning-based In-Painting for Virtual DSA", IEEE Nuclear Science Symposium and Medical Imaging Conference, 2017, vorgeschlagen wurde. Hierbei werden die durch Kontrastmittel überlagerten Hintergrundpixel mit Hilfe eines CNN (Convolutional Neural Network) unter Verwendung der U-Net-Architektur (vgl. hierzu auch den Artikel von Olaf Ronneberger et al., "U-Net: Convolutional Networks for Biomedical Image Segmentation", Medical Image Computing and Computer-Assisted Intervention (MICCAI) 9351, Seiten 234 - 241 (2015)) geschätzt.

Ersichtlich lassen sich "fehlende" Bildwerte für Pixel eines Projektionsbildes neben der Metallartefaktreduktion auch in einer Vielzahl anderer Anwendungsfälle sinnvoll ermitteln, wobei ein weiterer Anwendungsfall defekte Einzeldetektoren (Pixel) eines Röntgendetektors betreffen kann, so dass die Bestimmung bzw. Ersetzung von Bildwerten einzelner Pixel eines Projektionsbildes im Allgemeinen auch als "Defect Pixel Estimation" (DPE) bezeichnet werden kann. Die zur Ermittlung der Bildwerte verwendeten Verfahren werden auch als In-Painting-Verfahren bezeichnet.

In einem Artikel von Andre Aichert et al., "Epipolar Consistency in Transmission Imaging", IEEE Transactions on Medical Imaging 34 (2015), Seiten 2205 - 2219, wurden als mathematische Beschreibung von Redundanzen in den Projektionsbildern entsprechend der Projektionsgeometrien die epipolaren Konsistenzbedingungen (Epipolar Consistency Conditions - ECC) hergeleitet. Nach einer linearen Transformation (Bedingungstransformation), die durch eine Radontransformation gefolgt von einer Ableitung entlang der Distanzkoordinate gebildet wird, müssen bestimmte Transformationswerte in zwei aus Projektionsbildern entstehenden Transformationsbildern gleich sein. Dabei können die Koordinatenpaare der jeweils gleichen Transformationswerte für die epipolaren Konsistenzbedingungen aus den Projektionsgeometrien, die insbesondere durch Projektionsmatrizen beschrieben sein können, berechnet werden. Bei korrekter Aufnahme von Projektionsbildern eines Untersuchungsbereichs sind die epipolaren Konsistenzbedingungen allesamt erfüllt. Bezüglich der epipolaren Konsistenzbedingungen wurde bereits vorgeschlagen, eine Bewegungskorrektur zu realisieren, indem Verschiebungen von übereinstimmenden Werten in den Transformationsbildern gesucht wurden.

DE 10 2017 200 282 B3 offenbart ein Verfahren zu Reduktion von Bildartefakten, bei dem ein in seinen Parametern lineares Korrekturmodell auf die Projektionen angewandt werden soll, um artefaktreduzierte Bilddaten zu erhalten. Die Parameter des Korrekturmodells werden unter Berücksichtigung von von den Parametern unabhängigen Datentermen, welche eine auf den Epipolarlinien geltende Konsistenzbedingung der zumindest zwei Projektionen quantifizieren, durch Optimierung bestimmt. Mit anderen Worten werden dort aufgrund der epipolaren Konsistenzbedingungen Parameter eines Korrekturmodells bestimmt.

Der Erfindung liegt die Aufgabe zugrunde, eine Möglichkeit zum hochqualitativen Ergänzen bzw. Ersetzen von Bildwerten bei In-Painting-Verfahren anzugeben.

Zur Lösung dieser Aufgabe sind bei einem Verfahren der eingangs genannten Art erfindungsgemäß die Merkmale des Anspruchs 1 vorgesehen.

Im Rahmen der vorliegenden Erfindung hat sich gezeigt, dass bei herkömmlichen In-Painting-Verfahren, beispielsweise bei den beschriebenen Interpolationsansätzen und/oder bei auf künstliche Intelligenz basierenden Ansätzen, die epipolaren Konsistenzbedingungen, die sich aus den Projektionsgeometrien der jeweils betrachteten Projektionsbilderpaare ergeben, meist als nicht erfüllt angesehen werden können, so dass die abgeschätzten Bildwerte, beispielsweise im Rahmen einer Metallartefaktreduktion, diese natürliche Konsistenz nicht zeigen und als von eher niedriger Qualität bewertet werden können. Daher wird vorgeschlagen, die epipolaren Konsistenzbedingungen (ECC) bereits bei der Ermittlung der zu ermittelnden Bildwerte für entsprechend markierte Pixel heranzuziehen, um die diesbezügliche Konsistenz möglichst weitgehend sicherzustellen und so die Qualität der abgeschätzten Bildwerte für die markierten Pixel zu erhöhen. Die epipolaren Konsistenzbedingungen beschreiben letztlich die Konsistenz der approximierten Bildwerte im Radonraum mit den anderen Projektionsbildern. Mehrere epipolare Konsistenzbedingungen, die bei der Ermittlung der zu ermittelnden Bildwerte der markierten Pixel genutzt werden sollen, können dabei als ein lineares Gleichungssystem formuliert werden, nachdem die Bedingungstransformation, die sich aus der Radontransformation und der Ableitung entlang der Distanzkoordinaten zusammensetzt, eine lineare Transformation darstellt. Das entsprechend entstehende lineare Gleichungssystem kann, wie noch näher erläutert werden wird, unmittelbar zum Erhalten konsistenter Bildwerte gelöst werden oder aber als zusätzliche Randbedingung/ zusätzliches Optimierungsziel mit anderen In-Painting-Verfahren kombiniert werden bzw. diesen hinzugefügt werden, um deren Abschätzungsqualität zu verbessern. Bevorzugt bezieht sich das lineare Gleichungssystem, wie noch dargelegt werden wird, auf die Projektionsbilder, mithin die zu bestimmenden und teilweise bekannten Bildwerte.

Es wurde erkannt, dass das Ersetzen defekter Bildwerte bzw. das Abschätzen und Hinzufügen fehlender Bildwerte markierter Pixel in Projektionsbildern durch In-Painting-Verfahren eine Vielzahl von Transformationswerten in den Transformationsbildern beeinflusst, wobei das Verwenden einer üblichen In-Painting-Methode mit hoher Wahrscheinlichkeit keine die epipolaren Konsistenzbedingungen erfüllenden Bildwerte produziert. Durch Berücksichtigung der epipolaren Konsistenzbedingungen bei der Ermittlung von Bildwerten für die markierten Pixel in wenigstens einem Projektionsbild des Projektionsbildersatzes wird jedoch die Konsistenz, soweit möglich, gewahrt. Insbesondere die Kombination von In-Painting-Verfahren wie Interpolation und/oder Ermittlungsalgorithmen der künstlichen Intelligenz vermeidet inkonsistente Schätzungen und damit potentielle Artefakte in den rekonstruierten dreidimensionalen Bilddatensätzen.

Dabei können die epipolaren Konsistenzbedingungen im Allgemeinen einfach und recheneffizient zur konsistenten Schätzung von zu ermittelnden Bildwerten eingesetzt werden. Die Bildqualität des dreidimensionalen Bilddatensatzes wird erhöht und Artefakte können vermieden bzw. reduziert werden.

Dabei handelt es sich bei den Projektionsbildern insbesondere um medizinische, mit einer Röntgeneinrichtung, beispielsweise einer Röntgeneinrichtung mit einem C-Bogen oder einer Computertomographieeinrichtung, aufgenommene Bilddaten eines Patienten, die einen entsprechend aufzunehmenden Untersuchungsbereich des Patienten zeigen. Jedoch lässt sich das Verfahren auch auf andere, insbesondere Röntgenbildgebung nutzende Bildgebungstechniken bzw. Einsatzgebiete anwenden, beispielsweise die Materialprüfung oder dergleichen.

Vorzugsweise können als zu ermittelnde Bildwerte solche von markierten Pixeln in Metallbereichen in Projektionsbildern zeigenden Bildbereichen verwendet werden. In diesem Fall handelt es sich bei dem erfindungsgemäßen Verfahren mithin um ein Verfahren zur Metallartefaktreduktion, bei dem Metallobjekte im Untersuchungsbereich, der durch die Projektionsbilder des Projektionsbildersatzes gezeigt wird, letztlich "herausgerechnet" werden sollen, indem die entsprechenden, Metalle zeigenden markierten Pixel bzw. genauer deren Bildwerte durch approximierte, neue Bildwerte ersetzt werden sollen. Die entsprechenden, zu markierenden Pixel in den Projektionsbildern können dabei beispielsweise durch Segmentierung von Metallobjekten in einem vorläufig rekonstruierten dreidimensionalen Bilddatensatz und anschließende Vorwärtsprojektion in den Projektionsgeometrien aufgefunden werden, wobei auch andere Herangehensweisen grundsätzlich denkbar sind. Unter markiert soll dabei eine wie auch immer geartete Kennzeichnung oder Angabe verstanden werden, die es gestattet, die markierten Pixel als solche zu identifizieren bzw. aufzufinden. Bei den zu markierenden Pixeln kann es sich beispielsweise um fehlerhafte Bildwerte handeln, die als Metallartefakte verursacht werden. Über die Metallartefaktreduktion hinaus lässt sich das erfindungsgemäße Vorgehen selbstverständlich immer dann einsetzen, wenn Bildwerte markierter Pixel ersetzt werden sollen oder nicht vorliegen und daher abgeschätzt werden sollen, wie es beispielsweise auch bei defekten Pixeldetektoren eines Röntgendetektors vorkommen kann.

Eine Veränderung bzw. Hinzufügung eines Bildwerts an einem (markierten) Pixel muss nicht immer zwangsläufig einen nennenswerten Einfluss auf jede der sich aus den Projektionsgeometrien ergebenden epipolaren Konsistenzbedingungen haben. Zudem wäre die Kenntnis des Einflusses nützlich für die Aufstellung eines auf die Projektionsbilder bezogenen linearen Gleichungssystems. Daher sieht die vorliegende Erfindung vor, dass für jede sich aus den Projektionsgeometrien ergebende zu fordernde Übereinstimmung in zwei Transformationsbildern als einzelne epipolare Konsistenzbedingung deren Relevanz für die markierten Pixel in den Projektionsbildern anhand einer Relevanzbedingung überprüft wird, wobei nur relevante epipolare Konsistenzbedingungen, ein lineares Gleichungssystem bildend, bei der Ermittlung der zu ermittelnden Bildwerte verwendet werden. In einer konkreten, bevorzugten Ausgestaltung kann dabei vorgesehen sein, dass an jedem Pixel eines zu ermittelnden Bildwerts, mithin jedem markierten Pixel, eine Impulsfunktion angesetzt wird und die Bedingungstransformation auf das so entstehende und ansonsten leere Testbild angewendet wird, wobei die entstehenden Testtransformationsbilder für jedes Projektionsbild aufsummiert werden und als Relevanzbedingung überprüft wird, ob an für eine epipolare Konsistenzbedingung auszuwertenden Pixeln des Summenbildes der Testtransformationsbilder ein einen Grenzwert überschreitender Testtransformationssummenwert vorliegt, und/oder wobei die durch das Testtransformationsbild gegebene Impulsantwort zur Aufstellung des linearen Gleichungssystems verwendet wird. Mit anderen Worten wird die Bedingungstransformation auf Impulsfunktionen angewandt, die jeweils einzeln an den markierten Pixeln platziert werden, beispielsweise Delta-Funktionen. Diese können sich beispielsweise in Testbildern äußern, in denen nur am zu betrachtenden markierten Pixel (welches im Übrigen auch als defektes Pixel bezeichnet werden kann) der Wert 1 vorliegt, sonst der Wert 0. Die jeweilige Impulsantwort - das Testtransformationsbild - beschreibt jeweils den Einfluss des jeweiligen Bildwerts an dem entsprechenden markierten Pixel auf die Transformationswerte des Transformationsbildes, mithin eine Gewichtung, die zur Bildung des linearen Gleichungssystems genutzt werden kann. Nachdem üblicherweise Bildwerte für mehrere markierte Pixel innerhalb jedes Projektionsbildes bestimmt werden sollen, beispielsweise in einem Metallbereich, wird zudem vorgeschlagen, die Summe aller dieser Impulsantworten, also der Testtransformationsbilder, zu ermitteln, woraus sich eine binäre Maske für die Transformationsbilder durch Grenzwertvergleich mit dem Absolutbetrag der Summe bilden lässt. Der Grenzwert kann dabei beispielsweise heuristisch bestimmt werden und/oder von der Zahl der markierten Pixel in einem Projektionsbild abhängig sein. Die binäre Maske gibt die Pixel des Transformationsbildes an, deren Transformationswerte von den Bildwerten der markierten Pixel signifikant beeinflusst werden.

Dies ermöglicht es wiederum, jene epipolaren Konsistenzbedingungen auszuwählen, die tatsächlich eine Relevanz hinsichtlich der zu ermittelnden Bildwerte in den markierten Pixeln besitzen. Auf diese Weise können weniger oder überhaupt nicht relevante epipolare Konsistenzbedingungen der Betrachtung entzogen werden, so dass eine effiziente und aufwandsarme weitere Abschätzung der zu ermittelnden Bildwerte erfolgen kann.

Dabei sei darauf hingewiesen, dass in dem (in der Praxis eher selten auftretenden) Spezialfall, dass nur für ein Projektionsbild des Projektionsbildersatzes markierte Pixel vorliegen, grundsätzlich für jede (relevante) epipolare Konsistenzbedingung "korrekte" Transformationswerte aus anderen Projektionsbildern vorliegen. In dem Fall jedoch, in dem in mehreren (oder gar allen) Projektionsbildern markierte Pixel vorhanden sind, beispielsweise bei Abbildung eines Metallobjekts im Untersuchungsbereich, wird es häufig so sein, dass andere zu ermittelnde Bildwerte anderer Projektionsbilder einen Einfluss auf einen aus einem Projektionsbild hervorgehenden, für eine epipolare Konsistenzbedingung relevanten Transformationswert benötigten Vergleichs-Transformationswert haben, so dass ein deutlich komplexeres lineares Gleichungssystem entsteht. Das bedeutet, nicht für jeden Transformationswert, der von einem zu ermittelnden Bildwert beeinflusst wird, lässt sich zwangsläufig ein von zu ermittelnden Bildwerten unbeeinflusster Vergleichs-Transformationswert der entsprechenden epipolaren Konsistenzbedingung herleiten.

Wie bereits erwähnt, ist der große Vorteil bei der Betrachtung von Impulsantworten für einzelne markierte Pixel das entstehende Wissen um den Beitrag, den das markierte Pixel zu möglicherweise Teil einer epipolaren Konsistenzbedingung bildenden Transformationswerten liefert. Zur Erstellung des linearen Gleichungssystems im Raum der Projektionsbilder bei als Testtransformationsbildern vorliegenden Impulsantworten wird also bevorzugt für jedes Projektionsbild mit markierten Pixeln eine den Einfluss der (ja zu ermittelnden) Bildwerte der jeweiligen markierten Pixel auf die in den jeweils zu verwendenden, insbesondere also relevanten, epipolaren Konsistenzbedingungen enthaltenen Transformationswerte beschreibende Matrix ermittelt und zur Formulierung des linearen Gleichungssystems verwendet.

Im Rahmen der vorliegenden Erfindung ist es grundsätzlich möglich, dass die zu ermittelnden Bildwerte durch Lösung des durch die zu verwendenden epipolaren Konsistenzbedingungen gebildeten Gleichungssystems ermittelt werden. Sollen insgesamt eher wenige Bildwerte für markierte Pixel ermittelt werden, beispielsweise bei "defekten" Pixeln nur in einem oder sehr wenigen Projektionsbildern des Projektionsbilddatensatzes, kann durch die epipolaren Konsistenzbedingungen ein lineares Gleichungssystem entstehen, das lösbar oder sogar überbestimmt ist. Gerade aber in häufig in der Praxis auftretenden Anwendungsgebieten, beispielsweise bei der Metallartefaktreduktion, treten in allen Projektionsbildern des Projektionsbilddatensatzes markierte Pixel und somit zu ermittelnde Bildwerte auf, so dass in dem linearen Gleichungssystem auf beiden Seiten unbekannte Bildwerte stehen können, was das lineare Gleichungssystem mit höherer Wahrscheinlichkeit unterbestimmt macht. Auch für derartige unterbestimmte lineare Gleichungssysteme existieren jedoch noch Lösungsverfahren, um eine gutartige, sinnvolle Lösung in Form von zu ermittelnden Bildwerten auffinden zu können.

So kann beispielsweise vorgesehen sein, dass, insbesondere bei einem unterbestimmten Gleichungssystem, eine Tikhonov-Regularisierung und/oder wenigstens eine Vorwissen über die zu ermittelnden Bildwerte nutzende Randbedingung verwendet wird. Bei der bekannten Tikhonov-Regularisierung kann über die Wahl der Tikhonov-Matrix eine Vorgabe von Eigenschaften der erhaltenen Lösung mit in das lineare Gleichungssystem eingebracht werden. Beispielsweise ist es denkbar, eine Lösung mit geringer Energie und/oder eine Lösung mit wenig zusätzlichen Kanten anzustreben. Selbstverständlich ist es neben der Tikhonov-Regularisierung auch denkbar, andere Verfahren zu nutzen, um Randbedingungen einzubringen, die letztlich Hintergrundwissen über das vorhandene oder gewollte Verhalten innerhalb zu ersetzender Bereiche markierter Pixel beschreiben.

Nichtsdestotrotz hat es sich im Rahmen der vorliegenden Erfindung als bevorzugter Ansatz erwiesen, die epipolaren Konsistenzbedingungen als Randbedingung und/oder Optimierungsziel einem grundsätzlich bekannten bzw. üblichen In-Painting-Verfahren hinzuzufügen, so dass eine Ausgestaltung der vorliegenden Erfindung vorsieht, dass die zu verwendenden epipolaren Konsistenzbedingungen als Randbedingungen und/oder Optimierungsziel für wenigstens einen Ermittlungsalgorithmus zur Ermittlung der zu ermittelnden Bildwerte verwendet werden. In diesem Fall wird also ein Ermittlungsalgorithmus eingesetzt, der nicht das lineare Gleichungssystem löst, sondern auf anderem Wege versucht, Bildwerte der markierten Pixel abzuschätzen. Dabei kann der Ermittlungsalgorithmus beispielsweise auf eine Interpolation und/oder auf die Nutzung von künstlicher Intelligenz abzielen.

In einer bevorzugten Ausgestaltung der vorliegenden Erfindung kann vorgesehen sein, dass als der -Ermittlungsalgorithmus ein Algorithmus der künstlichen Intelligenz verwendet wird, welcher durch Maschinenlernen, insbesondere Deep Learning, trainiert ist. Gerade für Ermittlungsalgorithmen, die fehlende bzw. zu ersetzende Bildwerte in markierten Pixeln abschätzen sollen, ist es häufig sehr gut möglich, hochqualitative Trainingsdaten heranzuziehen, beispielsweise, indem im Fall der Metallartefaktreduktion ein Projektionsbildersatz erzeugt wird, der einen Gegenstand ohne Metallobjekte zeigt bzw. ein Untersuchungsbereich mit und ohne Metallobjekt aufgenommen wird. Somit steht dann die Grundwahrheit (ground truth) ohnehin zur Verfügung. Besonders vorteilhaft ist es in diesem Zusammenhang, wenn der Ermittlungsalgorithmus ein neuronales Netz, insbesondere ein CNN (Convolutional Neuronal Network) in U-Net-Architektur, umfasst. Dabei sei zunächst angemerkt, dass insbesondere im Fall von neuronalen Netzen sich das durch die epipolaren Konsistenzbedingungen ergebende lineare Gleichungssystem besonders einfach integrieren lässt, da jene eine lineare Gewichtung jeweils gefolgt von einem (optionalen) nichtlinearen Operator enthalten, was eine Einbindung des linearen Gleichungssystems in das neuronale Netz deutlich vereinfacht und einen direkten Nutzen mit sich bringt. Das bedeutet, der Einsatz der epipolaren Konsistenzbedingungen erscheint in einer Kombination mit auf maschinellem Lernen, insbesondere Deep Learning, basierenden In-Painting-Verfahren als besonders nützlich.

Dabei sei an dieser Stelle, auch unabhängig von der Nutzung von epipolaren Konsistenzbedingungen, angemerkt, dass die Verwendung von CNN, insbesondere in U-Net-Architektur, wie durch die eingangs zitierten Artikel von Unberath et al. und Ronneberger et al. beschrieben, einen äußerst nützlichen Ansatz zur Schätzung von zu ermittelnden Bildwerten darstellt, gerade auch bei lateral trunkierten Aufnahmen eines Untersuchungsbereichs eines Patienten. Bei der Verwendung solcher Ermittlungsalgorithmen der künstlichen Intelligenz bzw. speziell von CNNs kann ein Bildausschnitt um die markierten Pixel, beispielsweise ein Metallobjekt, mit den maskierten Bildwerten der markierten Pixel als Eingangsdaten verwendet werden, woraufhin der Ermittlungsalgorithmus ein Bild mit den geschätzten Bildwerten für die markierten Pixel als Ausgabe liefert. Die "Metallwerte" im Eingangsbild werden dann durch die geschätzten Bildwerte im Ausgangsbild ersetzt. Hierdurch können Bildwerte für verschiedenste Metallformen geschätzt werden, solange das Metallobjekt kleiner ist als der Bildausschnitt.

Im Rahmen der vorliegenden Erfindung kann ferner vorgesehen sein, dass der Ermittlungsalgorithmus eine interpolierende Bestimmung der zu ermittelnden Bildwerte vornimmt, insbesondere nach dem Verfahren der normalisierten Metallartefaktreduktion (NMAR). Dabei kann beispielsweise eine Vorwärtsprojektion eines initial korrigierten Volumens für eine verbesserte Interpolation herangezogen werden, wobei allerdings eine konsistente Schätzung gemäß der epipolaren Konsistenzbedingungen nicht garantiert wird. Insoweit kann die hier vorgeschlagene Nutzung der epipolaren Konsistenzbedingungen auch in Kombination mit der NMAR zu deutlich verbesserten Abschätzungen für markierte Pixel führen.

Das hier beschriebene Verfahren kann durch eine Recheneinrichtung durchgeführt werden, insbesondere aber auch durch eine Steuereinrichtung einer Röntgeneinrichtung, so dass an der Röntgeneinrichtung selbst bereits entsprechende Maßnahmen zum Ersetzen und/oder Ergänzen fehlerhafter, zu entfernender und/oder fehlender Bildwerte bereitgestellt werden kann.

Neben dem Verfahren betrifft die vorliegende Erfindung entsprechend auch eine Röntgeneinrichtung, aufweisend eine zur Durchführung des erfindungsgemäßen Verfahrens ausgebildete Steuereinrichtung. Sämtliche Ausführungen bezüglich des erfindungsgemäßen Verfahrens lassen sich analog auf die erfindungsgemäße Röntgeneinrichtung übertragen, mit welcher mithin ebenso die bereits genannten Vorteile erhalten werden können. Insbesondere kann es sich bei der Röntgeneinrichtung um eine Röntgeneinrichtung mit einem C-Bogen, an dem sich gegenüberliegend eine Röntgenquelle und ein Röntgendetektor angeordnet sind, handeln. Alternativ kann es sich bei der Röntgeneinrichtung um eine Computertomographieeinrichtung handeln, in der beispielsweise wenigstens ein Paar von Röntgenquelle und Röntgendetektor innerhalb einer Gantry um den Patienten bewegt werden kann. Die Steuereinrichtung einer solchen Röntgeneinrichtung kann nun eingerichtet sein, auf die erfindungsgemäße Art Bildwerte markierter Pixel abzuschätzen, was bereits an der Röntgeneinrichtung nützliche, hochqualitative Funktionen zur Erhöhung der Bildqualität aufgenommener Bilddaten und insbesondere dreidimensionaler Bilddatensätze bereitstellt. Die Steuereinrichtung kann wenigstens einen Prozessor und/oder wenigstens ein Speichermittel aufweisen und/oder eine Ermittlungseinheit umfassen, um zu ermittelnde Bildwerte unter Verwendung von epipolaren Konsistenzbedingungen zu ermitteln.

Ein erfindungsgemäßes Computerprogramm ist beispielsweise direkt in einen Speicher einer Recheneinrichtung, beispielsweise einer Steuereinrichtung einer Röntgeneinrichtung, ladbar und weist Programmmittel auf, um die Schritte eins hierin beschriebenen Verfahrens auszuführen, wenn das Computerprogramm in der Recheneinrichtung ausgeführt wird. Das Computerprogramm kann auf einem erfindungsgemäßen elektronisch lesbaren Datenträger gespeichert sein, welcher mithin darauf gespeicherter elektronisch lesbare Steuerinformationen umfasst, welche zumindest ein erfindungsgemäßes Computerprogramm umfassen und derart ausgestaltet sind, dass sie bei Verwendung des Datenträgers in einer Recheneinrichtung, insbesondere einer Steuereinrichtung einer Röntgeneinrichtung, ein erfindungsgemäßes Verfahren durchführen. Bei dem Datenträger kann es sich um einen nichttransienten Datenträger, beispielsweise eine CD-ROM, handeln.

Weitere Vorteile und Einzelheiten der vorliegenden Erfindung ergeben sich aus den im Folgenden beschriebenen Ausführungsbeispielen sowie anhand der Zeichnung. Dabei zeigen:

Fig. 1     eine Skizze zur Erläuterung von epipolaren Konsistenzbedingungen,

Fig. 2     einen prinzipiellen Ablaufplan von Ausführungsbeispielen des erfindungsgemäßen Verfahrens,

Fig. 3     eine Skizze zur Erläuterung der Verwendung von Relevanzbedingungen,

Fig. 4     eine Skizze zur Verbesserung der Erfüllung von epipolaren Konsistenzbedingungen, und

Fig. 5     eine erfindungsgemäße Röntgeneinrichtung.

Im Folgenden werden Ausführungsbeispiele des erfindungsgemäßen Verfahrens im Hinblick auf die medizinische Bildgebung, konkret die Metallartefaktreduktion in zu rekonstruierenden dreidimensionalen Röntgen-Bilddatensätzen, dargestellt. Hierbei werden, wie grundsätzlich bekannt, beispielsweise entlang einer kreisförmigen Aufnahmetrajektorie, mittels einer Röntgeneinrichtung eine Vielzahl von Projektionsbildern eines Untersuchungsbereichs des Patienten unter Verwendung unterschiedlicher Projektionsgeometrien, die jeweils durch eine Projektionsmatrix beschrieben sind, aufgenommen. Diese Projektionsbilder bilden einen Projektionsbildersatz. Mittels der Projektionsbilder ist es nun grundsätzlich möglich, durch entsprechende Rekonstruktionsverfahren, beispielsweise die gefilterte Rückprojektion, einen dreidimensionalen Bilddatensatz aus den zweidimensionalen Projektionsbildern zu rekonstruieren. Liegen jedoch Metallobjekte im Untersuchungsbereich vor, kann dies zu Artefakten führen. Mithin können Metallobjekte und somit die Ursache für die Metallartefakte aus den Projektionsbildern herausgerechnet werden, indem entsprechende das Metallobjekt zeigende Bildwerte in entsprechend als das Metallobjekt zeigend markierten Pixeln der jeweiligen Projektionsbilder durch abgeschätzte Bildwerte ohne das Metallobjekt ersetzt werden. Die markierten Pixel, die beispielsweise aufgrund einer Segmentierung des Metallobjekts in einem vorläufig rekonstruierten dreidimensionalen Bilddatensatz ermittelt werden können, können dabei auch als "defekte" Pixel bezeichnet werden, für die ein (hier ersetzender) Bildwert aufgefunden werden soll. Im Rahmen der vorliegenden Erfindung werden, gegebenenfalls zusätzlich zu anderen, sogenannten In-Painting-Verfahren, zur Abschätzung der Bildwerte in den markierten Pixeln epipolare Konsistenzbedingungen berücksichtigt, wie sie im eingangs genannten Artikel von Aichert et al. beschrieben sind.

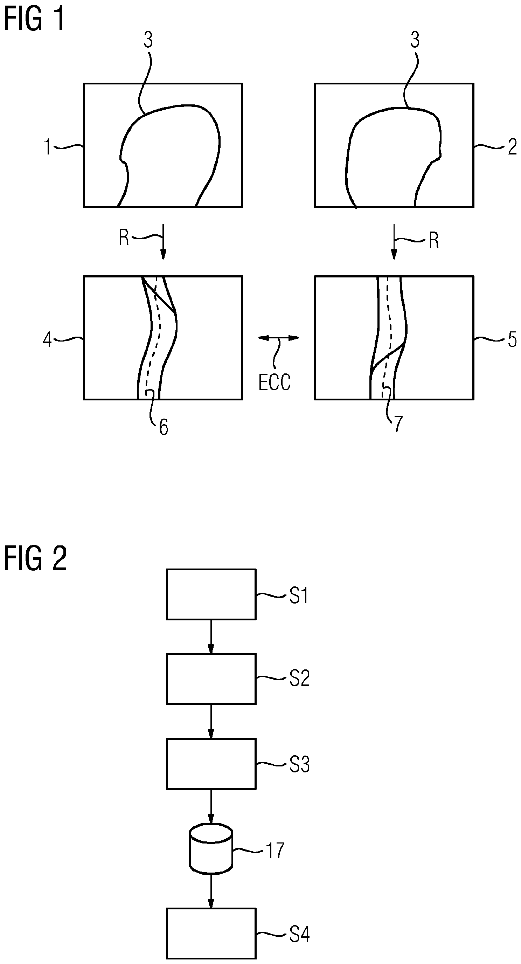

Zur Erläuterung der epipolaren Konsistenzbedingungen sind in Fig. 1 zwei unter unterschiedlichen Projektionsgeometrien aufgenommene Projektionsbilder 1, 2 eines Untersuchungsbereichs eines Patienten gezeigt, die, wie grundsätzlich bekannt, anatomische Strukturen 3 des Patienten aus unterschiedlichen Blickrichtungen zeigen. Werden die Kosinusgewichteten Bildwerte der Projektionsbilder 1, 2 als $\mathbf{p}_\lambda$ bezeichnet, wobei $\lambda$ einen laufenden Index der Projektionsbilder 1, 2 darstellt, ergibt sich die einem Transformationsbild 4, 5 entsprechende Grangeat'sche Zwischenfunktion $\mathbf{r}_\lambda$ als

$$\mathbf{r}_\lambda \;=\; R\mathbf{p}_\lambda \;,$$

wobei die im Folgenden als Bedingungstransformation bezeichnete Transformation **R** eine Radontransformation (diskretisiert) gefolgt von einer Ableitung entlang der Distanzkoordinate umfasst.

Sind nun, im hier dargestellten Beispiel über die Projektionsmatrizen, die Projektionsgeometrien der Projektionsbilder 1, 2 bekannt, lässt sich hieraus ableiten, dass Transformationswerte bestimmter Pixel der Transformationsbilder 4, 5 einander entsprechen müssen, in Formeln, wenn die entsprechenden Koordinaten der bestimmten Pixel als ECC (l, 1) für das Transformationsbild 4 und als ECC (l, 2) für das Transformationsbild 5 bezeichnet werden und k die Anzahl der epipolaren Konsistenzbedingungen ist:

$$\mathbf{r}_{\lambda 1}\,(ECC(l,\,1)) \;=\; \mathbf{r}_{\lambda 2}\,(ECC\,(l,\,2))$$

mit l = 1 .... k.

Die gestrichelten Linien 6, 7 der Fig. 1 können beispielsweise beschreiben, wo in den Transformationsbildern 4, 5 solche epipolaren Konsistenzbedingungen auftreten können. Mit anderen Worten entspricht die gestrichelte Linie 6 der Folge der Koordinaten ECC (l, 1), die gestrichelte Linie 7 der Folge der Koordinaten ECC (l, 2).

Unter perfekten Aufnahmebedingungen gelten die epipolaren Konsistenzbedingungen exakt. Werden nun jedoch in einem oder beiden der Projektionsbilder 1, 2, beispielsweise aufgrund eines Metallobjekts, in markierten Pixeln abgeschätzte Bildwerte eingesetzt, werden ohne Berücksichtigung der epipolaren Konsistenzbedingungen diese mit hoher Wahrscheinlichkeit nicht erfüllt sein. Andererseits beschreiben die epipolaren Konsistenzbedingungen jedoch die Konsistenz im Radonraum, so dass bei Ermittlung von abgeschätzten Bildwerten für markierte Pixel unter Verwendung der epipolaren Konsistenzbedingungen oder zumindest eines Teils der bestehenden epipolaren Konsistenzbedingungen, die relevant für die markierten Pixel sind, eine deutlich hochqualitativere Abschätzung mit geringerer Artefaktneigung durch die Ersetzung des Metallobjekts erreicht wird.

Fig. 2 zeigt hierzu einen generellen Ablaufplan von Ausführungsbeispielen des erfindungsgemäßen Verfahrens. Dabei soll zunächst bestimmt werden, welche epipolaren Konsistenzbedingungen, die sich für einen Projektionsbildersatz formulieren lassen, überhaupt für das gestellte Problem - Abschätzung von Bildwerten für bestimmte markierte ("defekte") Pixel in einigen oder allen Projektionsbildern 1, 2 des Projektionsbildersatzes - Relevanz aufweisen.

Dazu wird in einem Schritt S1 zunächst für jedes Projektionsbild eine Anzahl von Testbildern erzeugt, die der Anzahl der markierten Pixel in diesem Projektionsbild entspricht. Diese Testbilder entsprechen letztlich einer Impulsfunktion, die an jeweils einem markierten Pixel positioniert ist. Vorliegend werden die Testbilder so erzeugt, dass an dem markierten Pixel ein Wert von 1, sonst überall ein Wert von 0 vorliegt. Werden diese Testbilder nun der Bedingungstransformation **R** unterzogen, ergibt sich, auf welche Pixel des Transformationsbildes der Bildwert des entsprechenden markierten Pixels welchen Einfluss hat. Daher wird im Schritt S1 der Fig. 2 die Bedingungstransformation **R** auf die jeweiligen entsprechenden Testbilder angewendet, um Testtransformationsbilder zu erhalten.

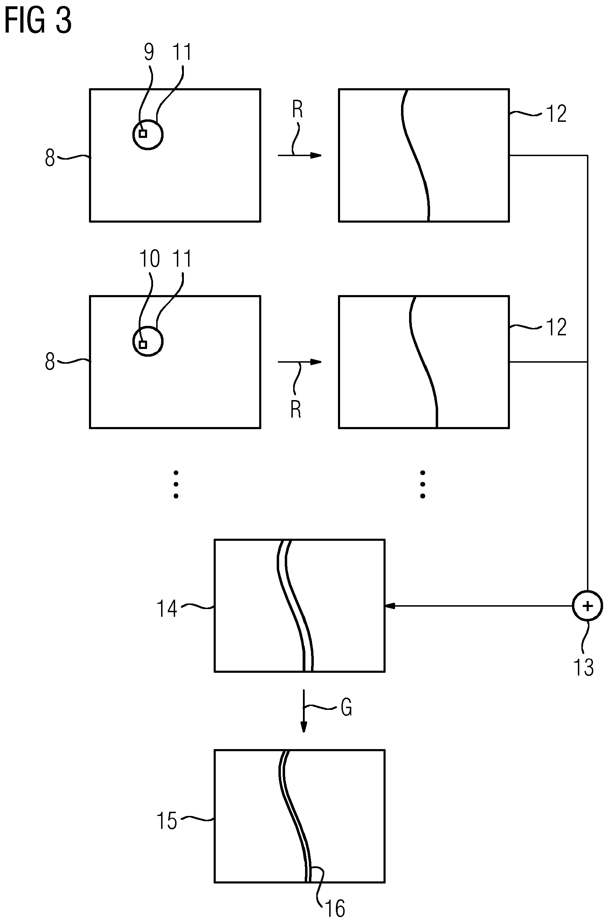

Dieser Vorgang wird durch Fig. 3 für eines der Projektionsbilder des Projektionsbildersatzes nochmals bildlich dargestellt. Zu sehen sind zunächst Testbilder 8, die sich jeweils auf ein unterschiedliches markiertes Pixel 9, 10 innerhalb des Bereichs 11 markierter Pixel eines bestimmten Projektionsbildes, beispielsweise eines der Projektionsbilder 1, 2, beziehen. Die Fortsetzungspunkte deuten an, dass entsprechende Testbilder 8 als Impulsfunktionen für alle markierten Pixel im Bereich 11 erzeugt werden. Auf diese Testbilder 8 wird dann jeweils die Bedingungstransformation **R** angewendet, um Testtransformationsbilder 12 zu erhalten.

Um eine Aussage zum Einfluss aller dieser markierten Pixel 9, 10 auf allgemeine Transformationsbilder zu erhalten, werden in einem Schritt S2 gemäß Fig. 2 für jedes der Projektionsbilder die entsprechenden entstehenden Testtransformationsbilder 12 aufsummiert, wie in Fig. 3 durch die Operation 13 dargestellt ist. Ergebnis ist ein Summenbild 14, auf das, weiterhin im Schritt S2, ein Grenzwertvergleich mit einem Grenzwert G angewandt wird, um eine binäre Maske 15 zu erzeugen. Der Grenzwert G kann von der Zahl der markierten Pixel in dem jeweiligen Projektionsbild abhängen und/oder heuristisch bestimmt werden. Die binäre Maske 15 gibt im Wesentlichen an, auf welche Transformationswerte Bildwerte der markierten Pixel 9, 10 einen relevanten Einfluss haben.

Damit kann die binäre Maske 15 in einem Schritt S3 genutzt werden, um relevante epipolare Konsistenzbedingungen aufzufinden. Hierzu werden Koordinaten von Pixeln in den Transformationsbildern 4, 5 jeweiliger Paare von Projektionsbildern 1, 2, für die epipolare Konsistenzbedingungen formuliert werden können, ermittelt und die binäre Maske 15 wird mit diesen Koordinaten (ECC(l, 1/2)) verknüpft. Es werden also die epipolaren Konsistenzbedingungen ausgewählt, für die wenigstens eine der entsprechenden Koordinaten innerhalb des durch die binäre Maske angezeigten Relevanzbereichs 16 (vgl. Fig. 3) liegen.

Diese relevanten epipolaren Konsistenzbedingungen gemeinsam mit den als Testtransformationsbilder 12 ermittelten Impulsantworten werden nun verwendet, um als Ergebnis der Schritte S1 bis S3 ein lineares Gleichungssystem 17 (vgl. Fig. 2) zu bilden, das in einem folgenden Schritt S4 bei der Ermittlung der Bildwerte für die markierten Pixel 9, 10 verwendet wird.

Zur Bildung des linearen Gleichungssystems 17 werden die vorteilhaft bekannten Einflüsse der einzelnen markierten Pixel 9, 10 auf die (relevanten) Transformationswerte für jedes Transformationsbild genutzt, um das lineare Gleichungssystem unmittelbar für die zu ermittelnden Bildwerte zu formulieren. Dabei werden für jedes Projektionsbild, in dem markierte Pixel 9, 10 vorliegen, Matrizen erstellt, die den Einfluss auf die in den relevanten epipolaren Konsistenzbedingungen enthaltenen Transformationswerte beschreiben.

Dies sei zur Verdeutlichung an einem einfachen Beispiel genauer dargelegt, in dem nur in einem Projektionsbild markierte Pixel 9, 10 vorliegen, alle anderen jedoch vollständig bekannt sind. Das bedeutet, nur eine Seite der epipolaren Konsistenzbedingungen weist dann Unbekannte (also zu ermittelnde Bildwerte) auf.

Sei k die Anzahl der im Schritt S3 aufgefundenen relevanten epipolaren Konsistenzbedingungen und l die Zahl der markierten Pixel im Projektionsbild $\lambda 1$. Bezeichnet man ferner die Impulsantwort, also die Testtransformationswerte des zugehörigen Testtransformationsbildes 12 für ein markiertes Pixel v mit **IRS**$_v$, so sind die IRS jeweils m-dimensionale Vektoren (m=Gesamtzahl der Pixel). Definiert man nun die Matrix

$$\mathbf{W}(u,\ v) = \mathbf{IRS}_v(i_{u,1})\ ,$$

wo $i_{u,1}$ die Koordinaten des Pixels des Transformationsbildes aus dem Projektionsbild $\lambda 1$ beschreibt, an dem der Transformationswert der epipolaren Konsistenzbeding u abgelesen soll, so liegt diese Matrix **W** im k x l-dimensionalen Raum.

Definiert man nun ferner den Vektor

$$\mathbf{y}(u)\ =\ \mathbf{r}_{\lambda(u)}(i_{u,2})\ -\ \mathbf{r}_{\lambda 1}(i_{u,1})$$

im k-dimensionalen Raum, worin $\lambda(u)$ das Projektionsbild beschreibt, dass zur epipolaren Konsistenzbedingung u gehört, $i_{u,2}$ die Koordinate des entsprechenden Transformationswerts und **r** die Transformationswerte beschreibt, so beschreibt mithin **y**(u) die Abweichung von der Geltung der epipolaren Konsistenzbedingung u.

Der Lösungssatz **x** im l-dimensionalen Raum des linearen Gleichungssystems **W x = y** beschreibt dann die konsistenten zu ermittelnden Bildwerte.

Wenn, wie in den üblichen Fällen, in mehreren (oder allen) Projektionsbildern markierte Pixel 9,10 vorkommen, treten auf beiden Seiten der epipolaren Konsistenzbedingungen Unbekannte und entsprechende Matrizen **W** auf, so dass das Gleichungssystem 17 komplizierter als hier dargestellt, aber auf dieselbe Weise ermittelbar, ist.

Der Lösungssatz des linearen Gleichungssystems 17 beschreibt die zu ermittelnden Bildwerte der markierten Pixel 9, 10, die konsistent mit den jeweiligen anderen Projektionsbildern des Projektionsbildersatzes sind. Die Nutzung des linearen Gleichungssystems 17 kann auf unterschiedliche Art und Weise erfolgen.

In einer ersten Ausgestaltung ist es möglich, im Schritt S4 das lineare Gleichungssystem 17 zu lösen, wobei eine geeignete Lösung auch bei unterbestimmtem linearen Gleichungssystem 17 beispielsweise durch die Verwendung der Tikhonov-Regularisierung aufgefunden werden kann.

Besonders bevorzugt sind jedoch Ausführungsbeispiele, in denen die in dem linearen Gleichungssystem 17 enthaltenen epipolaren Konsistenzbedingungen als Randbedingung oder möglichst gut zu erfüllendes Optimierungsziel einem anderen In-Painting-Verfahren hinzugefügt werden. Während es dabei durchaus möglich ist, ein Interpolationsverfahren wie die NMAR (normalisierte Metallartefaktreduktion) zur Verwendung des linearen Gleichungssystems 17 zu erweitern, stellen Ermittlungsalgorithmen der künstlichen Intelligenz, insbesondere solche, die Convolutional Neural Networks (CNN) in U-Net-Architektur nutzen, eine besonders bevorzugte Ausführungsform dar. Denn aufgrund der Struktur neuronaler Netze lassen sich lineare Gleichungssysteme besonders einfach in diese integrieren. So entsteht eine besonders effiziente Umsetzung.

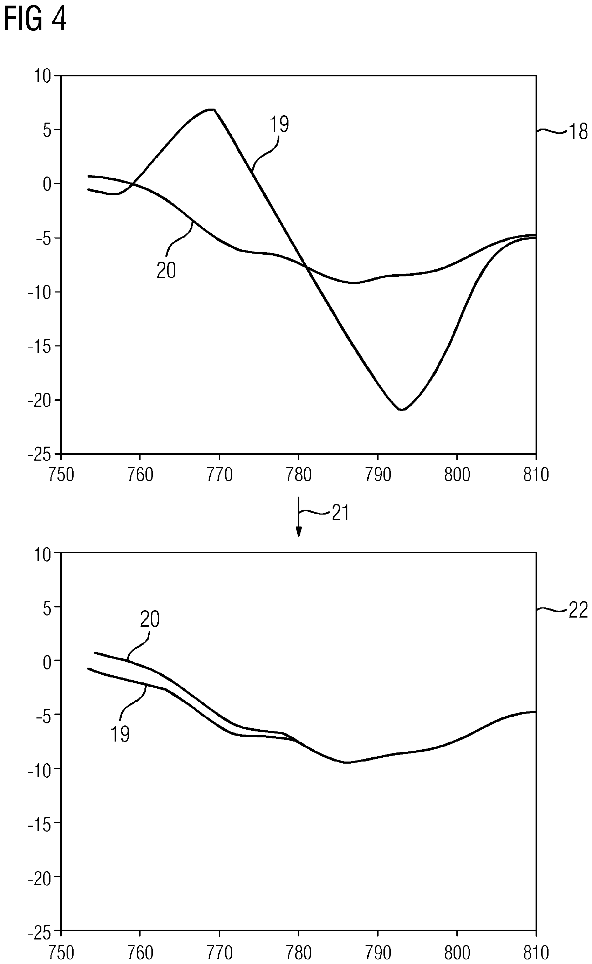

Fig. 4 zeigt beispielhaft die Entwicklung der Erfüllung von epipolaren Konsistenzbedingungen bei einer Nutzung derselben als ein Optimierungsziel. Der obere Graph 18 zeigt dabei die Verläufe 19, 20 von Transformationswerten, die sich gemäß relevanter epipolarer Konsistenzbedingungen entsprechen sollten. Ersichtlich existieren noch deutliche Unterschiede zwischen den einzelnen Transformationswerten der Verläufe 19, 20; es ist keine gute Konsistenz im Radonraum gegeben.

Gehen die epipolaren Konsistenzbedingungen, beispielsweise als das lineare Gleichungssystem 17, jedoch in einen Optimierungsprozess, angedeutet durch den Pfeil 21, eines Ermittlungsalgorithmus ein, wird eine deutlich bessere Konsistenz gemäß dem Graphen 22 erhalten, das bedeutet, die epipolaren Konsistenzbedingungen sind deutlich besser erfüllt. Dies kann beispielsweise dadurch erreicht sein, dass ein möglichst nahe am Lösungsraum des linearen Gleichungssystems 17 liegendes Tupel von Bildwerten für die markierten Pixel 9, 10 als Endergebnis verwendet wird oder das Endergebnis sogar innerhalb des Lösungsraums des linearen Gleichungssystems 17 liegt.

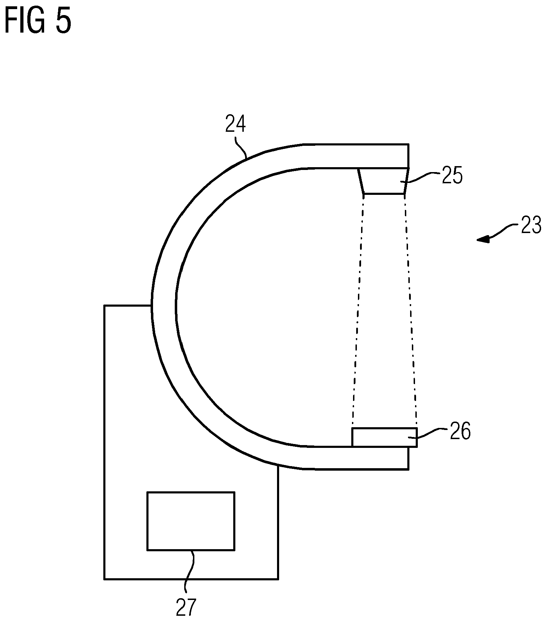

Fig. 5 zeigt schließlich schematisch eine Prinzipskizze einer erfindungsgemäßen Röntgeneinrichtung 23, die vorliegend als Röntgeneinrichtung 23 mit einem C-Bogen 24 realisiert ist, an dem sich gegenüberliegend eine Röntgenquelle 25 und ein Röntgendetektor 26 angeordnet sind. Der C-Bogen 24 ist beweglich und kann beispielsweise um einen Patienten herumgedreht werden, der auf einer hier nicht näher gezeigten Patientenliege gelagert ist. So können Projektionsbilder 1, 2 unter Verwendung unterschiedlicher Projektionsgeometrien erzeugt werden. Die Steuereinrichtung 27 der Röntgeneinrichtung 23 ist nun auch zur Durchführung des erfindungsgemäßen Verfahrens ausgebildet, das bedeutet, es können unmittelbar an der Röntgeneinrichtung 23 epipolare Konsistenzbedingungen bei der Abschätzung von Bildwerten für markierte Pixel verwendet werden. Die Steuereinrichtung 27 kann hierzu eine entsprechende Ermittlungseinheit sowie gegebenenfalls weitere Subeinheiten und/oder Funktionseinheiten aufweisen, um die unterschiedlichen Schritte des erfindungsgemäßen Verfahrens zu realisieren.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung, wie in den folgenden Patentansprüchen definiert, zu verlassen.

**Patentansprüche**

1.   Verfahren zur Ermittlung von Bildwerten in markierten Pixeln (9, 10) wenigstens eines Projektionsbildes (1, 2), wobei das wenigstens eine Projektionsbild (1, 2) Teil eines in einem Aufnahmevorgang jeweils in einer Projektionsgeometrie aufgenommene Projektionsbilder (1, 2) umfassenden, zur Rekonstruktion eines dreidimensionalen Bilddatensatzes vorgesehenen Projektionsbildersatzes ist, wobei die Ermittlung der Bildwerte unter Auswertung wenigstens einer wenigstens näherungsweise zu erfüllenden epipolaren Konsistenzbedingung, die sich aus den Projektionsgeometrien der unterschiedlichen Projektionsbilder (1, 2) des Projektionsbildersatzes ergibt und die Übereinstimmung zweier Transformationswerte in aus unterschiedlichen Projektionsbildern (1, 2) durch RadonTransformation und anschließende Ableitung als Bedingungstransformation ermittelten Transformationsbildern (4, 5) fordert, erfolgt, **dadurch gekennzeichnet, dass** für jede sich aus den Projektionsgeometrien ergebende zu fordernde Übereinstimmung in zwei Transformationsbildern (4, 5) als einzelne epipolare Konsistenzbedingung deren Relevanz für die markierten Pixel (9, 10) in den Projektionsbildern (1, 2) anhand einer Relevanzbedingung überprüft wird, wobei nur relevante epipolare Konsistenzbedingungen, ein lineares Gleichungssystem (17) bildend, bei der Ermittlung der zu ermittelnden Bildwerte verwendet werden.

2.   Verfahren nach Anspruch 1, **dadurch gekennzeichnet, dass** als zu ermittelnde Bildwerte solche von Metallbereiche in Projektionsbildern (1, 2) zeigenden Bildbereichen (11) verwendet werden.

3.   Verfahren nach Anspruch 1 oder 2, **dadurch gekennzeichnet, dass** an jedem markierten Pixel (9, 10) eine Impulsfunktion angesetzt wird und die Bedingungstransformation auf das so entstehende und ansonsten leere Testbild (8) angewendet wird, wobei die entstehenden Testtransformationsbilder (12) für jedes Projektionsbild (1, 2) aufsummiert werden und eine als Relevanzbedingung überprüft wird, ob an für eine epipolare Konsistenzbedingung auszuwertenden Pixeln des Summenbildes (14) der Testtransformationsbilder (12) ein einen Grenzwert überschreitender Testtransformationssummenwert vorliegt, und/oder die durch das Testtransformationsbild gegebene Impulsantwort zur Aufstellung des linearen Gleichungssystems verwendet wird.

4.   Verfahren nach einem der vorangehenden Ansprüche, **dadurch gekennzeichnet, dass** die zu ermittelnden Bildwerte durch Lösung des durch die zu verwendenden epipolaren Konsistenzbedingungen gebildeten Gleichungssystems (17) ermittelt werden.

5.   Verfahren nach Anspruch 4, **dadurch gekennzeichnet, dass**, insbesondere bei einem unterbestimmten Gleichungssystem (17), eine Tikhonov-Regularisierung und/oder wenigstens eine Vorwissen über die zu ermittelnden Bildwerte nutzende Randbedingung verwendet wird.

6.   Verfahren nach einem der vorangehenden Ansprüche, **dadurch gekennzeichnet, dass** die zu verwendenden epipolaren Konsistenzbedingungen als Randbedingungen und/oder Optimierungsziel für wenigstens einen Ermittlungsalgorithmus zur Ermittlung der zu ermittelnden Bildwerte verwendet werden.

7.   Verfahren nach Anspruch 6, **dadurch gekennzeichnet, dass** als der Ermittlungsalgorithmus ein Algorithmus der künstlichen Intelligenz verwendet wird, welcher durch Maschinenlernen trainiert ist.

8. Verfahren nach Anspruch 7, **dadurch gekennzeichnet, dass** der Ermittlungsalgorithmus ein neuronales Netz, insbesondere ein CNN in U-Net-Architektur, umfasst.

9. Verfahren nach einem der Ansprüche 6 bis 8, **dadurch gekennzeichnet, dass** der Ermittlungsalgorithmus eine interpolierende Bestimmung der zu ermittelnden Bildwerte vornimmt, insbesondere nach dem Verfahren der normalisierten Metallartefaktreduktion.

10. Röntgeneinrichtung (23), aufweisend eine zur Durchführung eines Verfahrens nach einem der vorangehenden Ansprüche ausgebildete Steuereinrichtung (27).

11. Computerprogramm, welches die Schritte eines Verfahrens nach einem der Ansprüche 1 bis 9 durchführt, wenn es auf einer Recheneinrichtung ausgeführt wird.

12. Elektronisch lesbarer Datenträger, auf dem ein Computerprogramm nach Anspruch 11 gespeichert ist.


**Claims**

1. Method for ascertaining image values in marked pixels (9, 10) of at least one projection image (1, 2), wherein the at least one projection image (1, 2) is part of a projection image set, which comprises projection images (1, 2) recorded during a recording procedure in a respective projection geometry and which is provided for reconstructing a three-dimensional image data record, wherein the ascertainment of the image values is implemented by evaluating at least one epipolar consistency condition, which should be at least approximately satisfied, which emerges from the projection geometries of the different projection images (1, 2) of the projection image set and which requires the correspondence of two transformation values in transformation images (4, 5), which are ascertained from different projection images (1, 2) by a Radon transform and the subsequent differentiation as the condition transformation, **characterized in that**, for each demanded correspondence in two transformation images (4, 5) arising from the projection geometries, the relevance thereof for the marked pixels (9, 10) in the projection images (1, 2) is checked on the basis of a relevance condition as the single epipolar consistency condition, with only relevant epipolar consistency conditions, which form a linear system of equations (17), being used when ascertaining the image values to be ascertained.

2. Method according to Claim 1, **characterized in that**, as image values to be ascertained, use is made of those of metal regions in image regions (11) exhibiting projection images (1, 2) .

3. Method according to Claim 1 or 2, **characterized in that** a pulse function is formulated at each marked pixel (9, 10) and the condition transformation is applied to the test image (8), which arises thus and is otherwise empty, wherein the arising test transformation images (12) are summed for each projection image (1, 2) and one is checked as a relevance condition as to whether the test transformation summed value that exceeds a limit is present at pixels of the summed image (14) of the test transformation images (12), which should be evaluated for an epipolar consistency condition, and/or the pulse response given by the test transformation image is used to set up the linear system of equations.

4. Method according to any one of the preceding claims, **characterized in that** the image values to be ascertained are ascertained by solving the system of equations (17) formed by the epipolar consistency conditions to be used.

5. Method according to Claim 4, **characterized in that** a Tikhonov regularization and/or at least one boundary condition using prior knowledge about the image values to be ascertained is used, particularly in the case of an underdetermined system of equations (17).

6. Method according to any one of the preceding claims, **characterized in that** the epipolar consistency conditions to be used are used as boundary conditions and/or optimization targets for at least one ascertainment algorithm for ascertaining the image values to be ascertained.

7. Method according to Claim 6, **characterized in that** an artificial intelligence algorithm, which is trained by machine learning, is used as the ascertainment algorithm.

**8.** Method according to Claim 7, **characterized in that** the ascertainment algorithm comprises a neural network, in particular a CNN in U-Net architecture.

**9.** Method according to any one of Claims 6 to 8, **characterized in that** the ascertainment algorithm carries out an interpolating determination of the image values to be ascertained, in particular according to the method of normalized metal artefact reduction.

**10.** X-ray device (23), comprising a control device (27) embodied to carry out a method according to any one of the preceding claims.

**11.** Computer program which, when executed on a computing device, carries out the steps of a method according to any one of Claims 1 to 9.

**12.** Electronically readable data medium, on which a computer program according to Claim 11 is stored.


**Revendications**

**1.** Procédé de détermination de valeurs d'image dans des pixels (9, 10) marqués d'au moins une image (1, 2) de projection, la au moins une image (1, 2) de projection étant une partie d'un ensemble d'images de projection comprenant des images (1, 2) de projection, enregistrées dans une opération d'enregistrement, respectivement, suivant une géométrie de projection, et prévue pour la reconstruction d'un ensemble de données d'image en trois dimensions, la détermination des valeurs d'image s'effectuant en exploitant au moins une condition de cohérence épipolaire à satisfaire au moins approximativement, qui provient des géométries de projection des images (1, 2) de projection différentes de l'ensemble d'images de projection et qui exige la coïncidence de deux valeurs de transformation en des images (4, 5) de transformation déterminées comme transformation de condition à partir d'images (1, 2) de projection différentes par transformation radon et dérivée ensuite,
**caractérisé en ce que**, pour chaque coïncidence à exiger provenant des géométries de projection dans deux images (4, 5) de transformation, on contrôle, à l'aide d'une condition de pertinence, comme condition de cohérence épipolaire individuelle, leur pertinence pour les pixels (9, 10) marqués dans les images (1, 2) de projection, seules des conditions de cohérence épipolaires pertinentes, formant un système (17) d'équations linéaire, étant utilisées lors de la détermination des valeurs d'image à déterminer.

**2.** Procédé suivant la revendication 1, **caractérisé en ce que**, comme valeurs d'image à déterminer, on utilise celles des parties (11 d'image présentant des parties métalliques dans des images (1, 2) de projection.

**3.** Procédé suivant la revendication 1 ou 2, **caractérisé en ce qu'**à chaque pixel (9, 10) marqué, on adjoint une fonction d'impulsion et on applique la transformation de condition à l'image (8) de test ainsi créée et sinon vide, les images (12) de transformation de test créées étant sommées pour chaque image (1, 2) de projection et on contrôle comme condition de pertinence, si, sur des pixels à exploiter pour une condition de cohérence épipolaire de l'image (14) somme des images (12) de tranformation de test, il y a une valeur somme de transformation de test dépassant une valeur limite et/ou on utilise la réponse impulsionnelle donnée par l'image de transformation de test pour l'établissement du système d'équations linéaire.

**4.** Procédé suivant l'une des revendications précédentes, **caractérisé en ce que** l'on détermine les valeurs d'image à déterminer en résolvant le système (17) d'équations formé par les conditions de cohérence épipolaires à utiliser.

**5.** Procédé suivant la revendication 4, **caractérisé en ce que**, notamment pour un système (17) d'équations sous-déterminé, on utilise une régularisation de Tikhonov et/ou au moins une prédiction sur la condition limite à utiliser pour des valeurs d'image à déterminer.

**6.** Procédé suivant l'une des revendications précédentes, **caractérisé en ce que** l'on utilise les conditions de cohérence épipolaires à utiliser comme conditions aux limites et/ou comme cible d'optimisation pour au moins un algorithme de détermination, afin de déterminer les valeurs d'image à déterminer.

**7.** Procédé suivant la revendication 6, **caractérisé en ce que** l'on utilise comme algorithme de détermination un algorithme de l'intelligence artificielle, qui subit un apprentissage par apprentissage automatique.

**8.** Procédé suivant la revendication 7, **caractérisé en ce que** l'algorithme de détermination comprend un réseau neuronal, notamment un CNN dans une architecteur U-Net.

**9.** Procédé suivant l'une des revendications 6 à 8, **caractérisé en ce que** l'algorithme de détermination effectue une détermination par interpolation des valeurs d'image à déterminer, notamment par le procédé de la réduction d'artéfact métallique normalisée.

**10.** Dispositif (23) à rayons X ayant un dispositif (27) de commande constitué pour effectuer un procédé suivant l'une des revendications précédentes.

**11.** Programme d'ordinateur, qui effectue les stades d'un procédé suivant l'une des revendications 1 à 9 lorsqu'il est réalisé sur un dispositif informatique.

**12.** Support de données déchiffrables électroniquement, sur lequel est mis en mémoire un programme d'ordinateur suivant la revendication 11.

FIG 1

FIG 2

# FIG 3

FIG 4

# FIG 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102017200282 B3


**In der Beschreibung aufgeführte Nicht-Patentliteratur**

- **ESTHER MEYER et al.** Normalized metal artifact reduction (NMAR) in computed tomography. *Medical Physics,* 2010, vol. 37, 5482
- **MATHIAS UNBERATH et al.** Deep Learning-based In-Painting for Virtual DSA. *IEEE Nuclear Science Symposium and Medical Imaging Conference,* 2017
- **OLAF RONNEBERGER et al.** U-Net: Convolutional Networks for Biomedical Image Segmentation. *Medical Image Computing and Computer-Assisted Intervention (MICCAI),* 2015, vol. 9351, 234-241
- **ANDRE AICHERT et al.** Epipolar Consistency in Transmission Imaging. *IEEE Transactions on Medical Imaging,* 2015, vol. 34, 2205-2219